(19) Europäisches Patentamt / European Patent Office / Office européen des brevets

(11) **EP 2 301 543 B1**

(12) **EUROPEAN PATENT SPECIFICATION**

(45) Date of publication and mention of the grant of the patent:
**29.07.2015 Bulletin 2015/31**

(51) Int Cl.:
**A61K 31/445** (2006.01)   **A61P 3/10** (2006.01)

(21) Application number: **08783943.7**

(22) Date of filing: **11.08.2008**

(86) International application number:
**PCT/CN2008/071950**

(87) International publication number:
**WO 2009/152665 (23.12.2009 Gazette 2009/52)**

(54) **A PHARMACEUTICAL COMPOSITION FOR TREATING DIABETES**

PHARMAZEUTISCHE ZUSAMMENSETZUNG ZUR BEHANDLUNG VON DIABETES

COMPOSITION PHARMACEUTIQUE POUR TRAITER LE DIABÈTE

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **20.06.2008 CN 200810031546**

(43) Date of publication of application:
**30.03.2011 Bulletin 2011/13**

(73) Proprietor: **Hill Pharmaceutical Co., Ltd Hunan 425000 (CN)**

(72) Inventors:
• **ZHOU, Yingjun**
  **Changsha**
  **Hunan 410008 (CN)**
• **ZENG, Guangyao**
  **Changsha**
  **Hunan 410013 (CN)**
• **JIANG, Meixiang**
  **Changsha**
  **Human 410007 (CN)**

(74) Representative: **Grünecker Patent- und Rechtsanwälte**
  **PartG mbB**
  **Leopoldstraße 4**
  **80802 München (DE)**

(56) References cited:
  WO-A1-2008/025249    CN-A- 1 559 539
  CN-A- 1 589 835      CN-A- 1 724 003
  JP-A- 9 140 351

• TANIGUCHI S ET AL: "Potentiation of glucose-induced insulin secretion by fagomine, a pseudo-sugar isolated from mulberry leaves", HORMONE AND METABOLIC RESEARCH, THIEME-STRATTON, STUTTGART, DE, vol. 30, no. 11, 1 November 1998 (1998-11-01), pages 679-683, XP009144449, ISSN: 0018-5043
• KIMURA MASAYASU ET AL: "Antihyperglycemic effects of N-containing sugars derived from mulberry leaves in streptozocin-induced diabetic mice", WAKAN IYAKUGAKU ZASSHI - JOURNAL OF TRADITIONAL MEDICINES, WAKAN IYAKU GAKKAI, TOYAMA, JP, vol. 12, no. 3, 1 January 1995 (1995-01-01), pages 214-219, XP008120663, ISSN: 1340-6302
• CHEN FU-JUN ET AL: "Potentiating effects on pilocarpine-induced saliva secretion, by extracts and N-containing sugars derived from mulberry leaves, in streptozocin-diabetic mice", BIOLOGICAL AND PHARMACEUTICAL BULLETIN, vol. 18, no. 12, 1995, pages 1676-1680, XP000002659189, ISSN: 0918-6158
• ALISON A. WATSON ET AL.: 'Polyhydroxylated alkaloids natural occurrence and therapeutic applications.' PHYTOCHEMISTRY vol. 56, 2001, pages 265 - 295, XP002331350
• NOJIMA H ET AL: "Antihyperglycemic effects of N-containing sugars from Xanthocercis zambesiaca, Morus bombycis, Aglaonema treubii, and Castanospermum australe in streptozotocin-diabetic mice", JOURNAL OF NATURAL PRODUCTS, AMERICAN CHEMICAL SOCIETY, US, vol. 61, no. 3, 1 March 1998 (1998-03-01), pages 397-400, XP002621629, ISSN: 0163-3864, DOI: 10.1021/NP970277L [retrieved on 1998-02-20]

Remarks:
    The file contains technical information submitted after the application was filed and not included in this specification

Remarks:
    The file contains technical information submitted after the application was filed and not included in this specification

**Description**

**FIELD OF INVENTION**

The invention relates to a pharmaceutical composition, in particular to a pharmaceutical composition for treating diabetes.

**BACKGROUND**

1-deoxynojirimycin and fagomine are nitrogen-containing alkaloids contained in branches, leaves, barks or silkworm droppings of moraceae plants. The chemical name of 1-deoxynojirimycin is 3,4,5-trihydroxy-2-hydroxymethyl tetrahydropyridine. The compound has been commercialized and can be purchased from the market, there are many manufacturers providing the compound, such as Shanghai Winherb Medical S & T Development Co., Ltd.

There is no public sale of commodities of the fagomine till now, and the literature ever reported that Naoki Asano, et al. in Hokuriku University, Japan obtained the fagomine through the method of extraction, repeated column chromatography and recrystallization ([J]. Carbohydrate Research, 253(1994)235-245).

Domestic and international researches and reports show that the alkaloid extracts from the moraceae plants have the roles of inhibiting the rise of blood glucose and improving glucose tolerance, wherein the 1-deoxynojirimycin can be combined with maltase, sucrase, lactase and other $\alpha$-glycosidases in small intestine, thereby avoiding further decomposition of disaccharides and further inhibiting the sharp rise in the postprandial blood glucose; and the fagomine not only has the role of a glycosidase inhibitor, but also has the role of reducing the blood glucose by increasing the release of insulin.

Hypoglycemic chemicals including sulfonylureas such as glyburide, gliclazide and the like, biguanides such as metformin and the like, thiazolidinediones such as troglitazone, rosiglitazone and the like have been commercialized and applied clinically; and flavonoids extract from mulberry leaves, ginkgo biloba extract compound, bitter melon extract and tea polyphenol extract and the like are on sale in the market, such as Hunan Geneham Biomedical Technology Ltd., Shaoguan Jumin Pharmaceutical Co., Ltd., and other medicine and extract

**SUMMARY OF INVENTION**

The invention aims at providing a pharmaceutical component with better effect on reducing blood glucose.

The composition contains 1-deoxynojirimycin and fagomine, wherein the weight ratio of the 1-deoxynojirimycin to the fagomine is 0.5 ~ 5:1.

Wherein, the preferential weight ratio of the 1-deoxynojirimycin to the fagomine is 2 ~ 5:1.

The invention firstly researches the role of reducing the blood glucose of the composition of the 1-deoxynojirimycin and the fagomine with different mixing ratios, and finds out that the two have good synergic hypoglycemic effect within a certain proportion range. According to the embodiments of the invention, the mixing ratio with better synergic hypoglycemic effect is selected.

The invention further researches the synergic hypoglycemic effect of the composition which is combined with other hypoglycemic compounds for use. On reducing the blood glucose value of rats with hyperglycemia after intragastric administration of sucrose, the joint application of the composition of the 1-deoxynojirimycin and the fagomine and other hypoglycemic substances can more effectively than other hypoglycemic substances or the combined application or the single application of the two.

The pharmaceutical composition containing the 1-deoxynojirimycin and the fagomine can be prepared into a variety of formulations, including tablets, capsules, granules, powder, dripping pills, oral liquid and the like. The preparation method can adopt the conventional excipients and the conventional method.

The research results show that the pharmaceutical composition of present application has clear action mechanism and significant efficacy and can be used for treating and preventing diabetes and complications thereof.

Description of the Preferred Embodiments

**Embodiment 1**

Determination of $\alpha$-D-glucosidase inhibitory activity of the combination compatibility of 1-deoxynojirimycin and fagomine.

1. Medicines: 1-deoxynojirimycin and fagomine, homemade.

2. Reagents: potassium dihydrogen phosphate, sodium hydroxide, sodium bicarbonate, para-nitrophenyl glucoside (PN-PG), reduced glutathione and $\alpha$-D-glucosidase sucrose.

3. Instrument: TU-1901 UV-Vis spectrophotometer.

4. Test method and results

(1) Preparation of potassium phosphate buffer solution: dissolve 0.68120g of potassium dihydrogen phosphate in 25mL of distilled water, obtain stock solution 1, dissolve 0.2g of NaOH in 25mL of the distilled water, obtain stock solution 2, suck the stock solution 1 (25mL), add into the stock solution 2 (11.8mL), shake up, dilute to 100mL and obtain the potassium phosphate buffer solution.

(2) $Na_2CO_3$ stop solution: $Na_2CO_3$ 10.6g → 100mL of distilled water (1mol/L)

(3) PNPG: 17.47mg → 2mL of distilled water (0,029mol/L)

(4) Reduced glutathione: 2.10mg → 2mL of distilled water (1.05mg/mL)

(5) $\alpha$-D-glucosidase sucrose: 0.5mg → 1mL of distilled water (0.5mg/mL)

(6) l-deoxynojirimycin stock solution: 3.24mg → 1mL of distilled water (20$\mu$ mol/mL)

(7) Fagomine stock solution: 3.00mg → 1mL of distilled water (20$\mu$ mol/mL)

(8) Preparation of glycosidase inhibitor sample solution:

Take 250uL of the 1-deoxynojirimycin stock solution, dilute to 1mL with the distilled water, and obtain the stock solution l.

Take 250$\mu$L of the fagomine stock solution, dilute to 1 mL with the distilled water, and obtain the stock solution 2.

The compatibility of the stock solution 1 and the stock solution 2 is carried out according to the proportion in Table 2.

(9) Determination reaction of $\alpha$-D-glucosidase inhibitory activity

Add the reagents according to the sequence in Table 1 for reaction, determine the absorbance value and calculate the $\alpha$-glycosidase inhibition ratio of a sample according to the following formula:

$$\alpha\text{-glycosidase inhibition ratio}\% = \frac{\text{Absorbance of sample tube-absorbance of blank tube}}{\text{Absorbance of standard tube-absorbance of blank tube}} \times 100\%$$

Table 1 Design of Determination Test of $\alpha$-glycosidase Inhibition Ratio

| Reagent ($\mu$L) | Blank centrifuge tube | Standard tube | Sample tube |
|---|---|---|---|
| Potassium phosphate buffer solution | 2500 | 2500 | 2500 |
| $\alpha$-D-glucosidase | 15 | 15 | 15 |
| Reduced glutathione | 15 | 15 | 15 |
| Distilled water | 45 | 30 | |
| Glycosidase inhibitor | | | 30 |
| Constant temperature for 10min in water bath cauldron at 37 C | | | |
| PNPG | | 15 | 15 |
| Constant temperature for 10min in water bath cauldron at 37 C | | | |
| $Na_2CO_3$ stop solution | 1425 | 1425 | 1425 |
| Colorimetry at UV 400nm | | | |

(10 Results: see Table 2.

Table 2 Test of $\alpha$-glycosidase Inhibition Ratio of 1-deoxynojirimycin and Fagomine in Different Mixing Ratios

| DNJ: Fag mixing ratio | 1:0 | 5:1 | 2:1 | 1:1 | 1:2 | 1:5 | 0:1 |
|---|---|---|---|---|---|---|---|
| DNJ: Fag sample solution volume | 30:0 | 25:5 | 20:10 | 15:15 | 10:20 | 5:25 | 0:30 |
| $\alpha$-glycosidase inhibition ratio% | 44% | 59% | 54% | 48% | 42% | 33% | 21% |
| Note: In the table, 1-deoxynojirimycin is called DNJ for short and fagomine is called Fag for short. | | | | | | | |

5. Conclusion: the test results show that the fagomine and the 1-deoxynojirimycin have the synergic $\alpha$-glycosidase inhibition role (the total amount of the 1-deoxynojirimycin and the fagomine is constant), when the mixing ratio of the 1-deoxynojirimycin to the fagomine is 0.5-5:1, the $\alpha$-glycosidase can be better inhibited, wherein when the mixing ratio is 2-5:1, the effect is obviously better than the effect of the single use of the 1-deoxynojirimycin in the same concentration.

**Embodiment 2**

Impacts of composition in different doses on blood glucose and serum insulin of mice with spontaneous hyperglycemia

1. Medicines: 1-deoxynojirimycin and fagomine, prepared by the inventor. The two are prepared into the required concentrations with distilled water before the test (the sum of the concentrations of the 1-deoxynojirimycin and the fagomine in the composition is the same as the concentration of the fagomine or the 1-deoxynojirimycin which is prepared alone). Glibenclamide, Hunan Dongting Pharmaceutical Co., Ltd.
2. Animals: NOD mice with the weight of 25-35g, provided by Experimental Animal Center, Peking University Health Science Center. Determine the blood glucose before the test and select the animals with the blood glucose value of above 11.1 mmol/L for carrying out the test.
3. Determination instrument: Yicheng blood glucose tester, developed by Beijing Yicheng Bioelectronic Technology Co., Ltd.
4. Main reagents: [125]I-insulin radioimmunoassay kit, produced by Department of Isotopes, China Institute of Atomic Energy.
5. Test method and results
(1) Impacts on the blood glucose of the mice: determine the blood glucose of the NOD mice after fasting for 6h, select 70 animals with the blood glucose of above 11.1 mmol/L, randomly divide into seven groups, respectively carry out intragastric administration and give the following medicines: i) control group (distilled water); ii) glibenclamide group (50mg/kg); and iii)-v) composition in different mixing ratios (20mg/kg, with different medicine proportions and constant total amount). Carry out the continuous administration for 14 days, fast for 6h before the administration at the 14th day, draw blood from the animals 1h after the administration, use the Yicheng blood glucose tester to determine the blood glucose value of the animals, and determine the serum insulin by radioimmunoassay. The results are shown in Tables 3-4.

Table 3 Impacts of Pharmaceutical Composition on Blood Glucose of Mice with Spontaneous Hyperglycemia (X±SD)

| Group | Dose (mg/kg) | Number of animal s | Blood glucose before administration (mmol/L) | Blood glucose after administration (mmol/L) |
|---|---|---|---|---|
| Model control group | --- | 10 | 13.64±2.08 | 13.97±1.86 |
| Glibenclamide | 50 | 10 | 13.26±2.26 | 10.57±2.17** |
| DNJ | 20 | 10 | 14.20±1.33 | 10.60±2.13** |
| Fagomine | 20 | 10 | 13.49±1.65 | 12.24±1.62** |
| Composition | 20 | 10 | 14.14±1.70 | 9.75±1.53** |
| Composition | 20 | 10 | 13.58±1.73 | 10.04±1.44** |
| Composition | 20 | 10 | 13.92±2.07 | 10.39±1.43** |

Note: 1. Compare with the model control group: *P<0.05, **P<0.01 (t-test).
2. In the table, l-deoxynojirimycin is called DNJ for short and fagomine is called Fag for short.

Table 4 Impacts of Pharmaceutical Composition on Serum Insulin of Mice with Spontaneous Hyperglycemia (X±SD)

| Group | Dose (mg/kg) | Number of animals | Serum insulin (MIU/L) |
|---|---|---|---|
| Model control group | ---- | 10 | 14.84±2.83 |
| Glibenclamide | 50 | 10 | 22.57±3.05** |
| 1-deoxynojirimycin | 20 | 10 | 16.14±2.63 |
| Fagomine | 20 | 10 | 21.78±3.34** |
| Composition (DNJ:Fag=5:1) | 20 | 10 | 17.25±2.15* |
| Composition (DNJ:Fag=2:1) | 20 | 10 | 19.83±2.65** |

(continued)

| Group | Dose (mg/kg) | Number of animals | Serum insulin (MIU/L) |
|---|---|---|---|
| Composition (DNJ:Fag=1:2) | 20 | 10 | 21.03±3.22** |

Note: 1. Compare with the model control group: *P<0.05, **P<0.01 (t-test).
2. In the table, 1-deoxynojirimycin is called DNJ for short and fagomine is called Fag for short.

6. Conclusion: The single application of the 1-deoxynojirimycin can significantly reduce the blood glucose of the NOD mice and has better effect than the single application of the fagomine for reducing the blood glucose; and the single application of the 1-deoxynojirimycin has few impacts on the serum insulin of the NOD mice, and the single application of the fagomine can significantly improve the serum insulin level;

wherein, when the mixing ratio of the 1-deoxynojirimycin to the fagomine is 2-5:1, the composition can improve the serum insulin level of the NOD mice and significantly reduce the blood glucose of the NOD mice, and the hypoglycemic effect is obviously better than the single application of the 1-deoxynojirimycin or the fagomine with the same concentration, representing that the combined application of the two has synergic effect.

In addition, according to the results of the efficacy test, the effective dose of the composition of the 1-deoxynojirimycin and the fagomine approximately equals to 120mg of the composition per person per day, with about 40mg each time; calculated by the preferential proportion of 2-5:1, each administration at least contains more than 25mg of the 1-deoxynojirimycin; from the aspects of facilitating the administration of patients and reducing the administration dose, tablets or capsules are adopted, and each administration contains 5 tablets or 5 capsules at most (the total amount is calculated as 2500mg at most), so that the preferential content of the 1-deoxynojirimycin in the pharmaceutical composition is larger than 1%.

**Embodiment 3**

Joint application test with other hypoglycemic substances
1. Medicines: 1-deoxynojirimycin-fagomine mixture (2:1), homemade. The mixture is prepared to the required concentration with distilled water before the test; glucobay, Bayer Healthcare Co., Ltd.; catechin, ginkgo biloba extract and flavonoids from mulberry leaves, Hunan Geneham Biomedical Technology Ltd.
2. Animals: SD rats with the weight of 180-220g, half male and half female, provided by Hunan Institute of Pharmaceutical Industry Research. Modeling method: carry out small dose intravenous injection of 25mg/kg of streptozotocin on the rats after fasting for 24h, once a week, simultaneously heat feed for feeding, induce 11 type diabetes, determine the blood glucose 1 week after the second injection, and select the animals with the fasting blood glucose of above 7.8mmol/L for carrying out the test.
3. Reagents: streptozotocin, product of Sigma Company, USA. Use citric acid buffer solution (PH=4.0) before modeling, and prepare at low temperature; sucrose, analytically pure.
4. Instrument: surestep plus blood glucose monitoring meter.
5. Test method and results: lead the SD rats to fast without water deprivation for 24h, carry out intravenous injection of 25mg/kg of streptozotocin, once a week, simultaneously heat feed for feeding, induce 11 type diabetes, determine the blood glucose 1 week after the second injection, and select 90 rats with the fasting blood glucose of above 7.8mmol/L for carrying out the test. Randomly divide into 9 groups (n = 10), wherein the first group is the control group with isometric distilled water for intragastric administration, the second group is the glucobay group, and other groups are the 1-deoxynojirimycin-fagomine mixture (the proportion of the mixture is 2:1, represented as DF in the table) and compositions of other different hypoglycemic substances; and carry out intragastric administration according to the above dose, give the test medicines continuously for 14 days, fast for 6h before the administration at 14th day, carry out the administration according to the above dose, simultaneously carry out the intragastric administration with 5.0g/kg of sucrose, and determine the blood glucose of the rats before sucrose administration and 1.0h after the sucrose administration, wherein the temperature in a laboratory is 20 °C-25°C. The test results are shown in Table 5.

Table 5 Impacts of Composition on Blood Glucose Value of Rats with Streptozotocin-induced Hyperglycemia, after Intragastric Administration with Sucrose

| Group | Medicine dose (mg/kg) | Blood glucose value of rats with hyperglycemia (mmol/L) | |
|---|---|---|---|
| | | Before administration | After administration |
| Model control group | Isometric distilled water | 8.45±0.82 | 15.56±2.54 |

(continued)

| Group | Medicine dose (mg/kg) | Blood glucose value of rats with hyperglycemia (mmol/L) | |
| --- | --- | --- | --- |
| | | Before administration | After administration |
| Glucobay group | 10.0 | 8.24±0.93 | 12.21±1.84** |
| DF: catechin (1:2) | 60 | 8.97±1.01 | 12.47±2.03** |
| Catechin | 60 | 9.17±0.89 | 14.10±1.50 |
| DF: ginkgo biloba extract (1:2) | 60 | 8.31±0.94 | 12.54±1.61** |
| Ginkgo biloba extract | 60 | 8.56±1.11 | 13.82±1.72 |
| DF: flavonoids from mulberry leaves | 60 | 7.96±1.07 | 12.04±1.91** |
| Flavonoids from mulberry leaves | 60 | 8.72±1.22 | 13.65±2.12* |
| DF | 20 | 8.37±1.05 | 12.88±1.55** |
| Note: Compare with the model control group: *P<0.05, **P<0.01. | | | |

6. Conclusion: The composition of the 1-deoxynojirimycin and the fagomine and other hypoglycemic substances can more effectively reduce the blood sugar value of the rats with the hyperglycemia after the intragastric administration of the sucrose in comparison with the combined and single applications of other hypoglycemic substances and DF.

**Claims**

1. A pharmaceutical composition for use in treating diabetes, wherein the composition contains 1-deoxynojirimycin and fagomine, and the weight ratio of the 1-deoxynojirimycin to the fagomine is 0.5-5:1.

2. The pharmaceutical composition for use according to claim 1, wherein, the weight percent of the 1-deoxynojirimycin is more than 1%.

3. The pharmaceutical composition for use according to claim 1, wherein, the preferential weight ratio of the 1-deoxynojirimycin to the fagomine is 2-5:1.

4. The pharmaceutical composition for use in treating diabetes of any one of claims 1-3, wherein the composition contains other compounds or extracts with hypoglycemic activity.

5. The pharmaceutical composition for use in treating diabetes of claim 4, wherein other compounds with the hypoglycemic activity are selected from chemicals of sulfonylureas, biguanides and thiazolidinediones; the extracts can be selected from mulberry leaf flavonoids separated and purified from moraceae plants, ginkgo biloba extract, bitter melon extract and tea polyphenol extract.

**Patentansprüche**

1. Eine pharmazeutische Zusammensetzung zur Behandlung von Diabetes, wobei die Zusammensetzung 1-Desoxynojirimycin und Fagomin enthält, und das Gewichtsverhältnis des 1-Desoxynojirimycin zu Fagomin 0,5-5:1 beträgt.

2. Die pharmazeutische Zusammensetzung zur Behandlung nach Anspruch 1, wobei der Gewichtsprozentsatz des 1-Desoxynojirimycin mehr als 1% beträgt.

3. Die pharmazeutische Zusammensetzung zur Behandlung nach Anspruch 1, wobei das bevorzugte Gewichtsverhältnis von 1-Desoxynojirimycin zu Fagomin 2-5:1 beträgt.

4. Die pharmazeutische Zusammensetzung zur Behandlung von Diabetes nach einem der Ansprüche 1-3, wobei die Zusammensetzung andere Verbindungen oder Extrakte mit hypoglykämischer Aktivität enthält.

**5.** Die pharmazeutische Zusammensetzung zur Behandlung von Diabetes nach Anspruch 4, wobei andere Verbindungen mit hypoglykämischer Aktivität aus Chemikalien der Sulfonylharnstoffe, Biguanide und Thiazolidindione ausgewählt werden; die Extrakte können aus Moraceae Pflanzen getrennten und aufgereinigten Maulbeerblatt-Flavonoiden, Ginkgo Biloba-Extrakt, Bittermelonenextrakt und Tee-Polyphenol-Extrakt ausgewählt werden.

**Revendications**

**1.** Composition pharmaceutique destinée à être utilisée dans le traitement du diabète, dans laquelle la composition contient de la 1-déoxynojirimycine et de la fagomine, et le rapport massique de la 1-déoxynojirimycine par rapport à la fagomine est compris entre 0,5:1 et 5:1.

**2.** Composition pharmaceutique destinée à une utilisation selon la revendication 1, dans laquelle le pourcentage massique de la 1-déoxynojirimycine est supérieur à 1 %.

**3.** Composition pharmaceutique destinée à une utilisation selon la revendication 1, dans laquelle le rapport massique préférentiel de la 1-déoxynojirimycine par rapport à la fagomine est compris entre 2:1 et 5:1.

**4.** Composition pharmaceutique destinée à être utilisée dans le traitement du diabète selon l'une quelconque des revendications 1 à 3, dans laquelle la composition contient d'autres composés ou extraits à activité hypoglycémique.

**5.** Composition pharmaceutique destinée à être utilisée dans le traitement du diabète selon la revendication 4, dans laquelle d'autres composés à activité hypoglycémique sont sélectionnés parmi des produits chimiques comprenant les sulfonylurées, les biguanides et les thiazolidinediones ; et les extraits peuvent être sélectionnés parmi les flavonoïdes de la feuille de mûrier séparés et purifiés à partir de plantes de la famille *Moraceae,* les extraits de *Ginkgo biloba,* les extraits de margose et les extraits de polyphénols du té.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NAOKI ASANO et al.** Hokuriku University, Japan obtained the fagomine through the method of extraction, repeated column chromatography and recrystallization ([J. *Carbohydrate Research,* 1994, vol. 253, 235-245